Europäisches Patentamt

European Patent Office

Office européen des brevets

(19)

(11) Veröffentlichungsnummer: **0 166 793**
**B1**

(12) # EUROPÄISCHE PATENTSCHRIFT

(45) Veröffentlichungstag der Patentschrift:
**02.11.88**

(51) Int. Cl.⁴: **A 61 M 1/00**

(21) Anmeldenummer: **84107609.4**

(22) Anmeldetag: **30.06.84**

(54) **Operationssauger.**

(43) Veröffentlichungstag der Anmeldung:
**06.01.86 Patentblatt 86/2**

(45) Bekanntmachung des Hinweises auf die Patenterteilung:
**02.11.88 Patentblatt 88/44**

(84) Benannte Vertragsstaaten:
**AT BE CH FR GB IT LI NL SE**

(56) Entgegenhaltungen:
**WO-A-84/04684**
**DE-A-3 307 517**
**DE-B-2 950 323**
**FR-A-1 426 230**
**US-A-3 335 727**
**US-A-3 911 919**
**US-A-4 212 300**

(73) Patentinhaber: **Härle, Anton, Dr., Drechslerweg 40,
D-4400 Münster- Roxel (DE)**

(72) Erfinder: **Härle, Anton, Dr., Drechslerweg 40,
D-4400 Münster- Roxel (DE)**

(74) Vertreter: **Habbel, Hans- Georg, Dipl.- Ing.,
Postfach 3429 Am Kanonengraben 11, D-4400
Münster (DE)**

EP 0 166 793 B1

LIBER, STOCKHOLM 1988

## Beschreibung

Die Erfindung betrifft einen Operationssauger gemäß dem Oberbegriff des Hauptanspruches.

In der DE-B-2 950 323, die eine gattungsgemäße Einrichtung gemäß dem Oberbegriff zeigt, wird ein Operationssauger beschrieben, bei welchem das Saugrohr aus zwei über ein Gehäuse zusammengesetzten Rohrstücken besteht und das eine, vorzugsweise schräg abgeschnittene Rohrende in dem Gehäuse einen Sitz für eine Ventilklappe bildet, die mittels eines die Gehäusewandung an einer elastischen Stelle durchsetzenden Stifts von dem Sitz abhebbar ist. Eine Kontaminierung der Saugrohrmündung durch die Bedienungsperson wird hierbei vermieden. Die Anordnung der gelenkigen Lagerung der Ventilklappe ist fertigungstechnisch schwierig und die Kraft zum Anheben der Ventilklappe relativ hoch, so daß die den Sauger bedienende Hand des Operateurs stark belastet wird.

Bei geschlossener Ventilklappe wirkt der im Saugrohrteil herrschende Unterdruck, der relativ groß ist, auf die Rückseite der Ventilklappe und zieht diese fest auf die Rohrmündung auf. Zum Anheben der Ventilklappe benötigt daher der Chirurg nicht nur die Kraft, um den Widerstand der elastischen Gelenklagerung zu überwinden, sondern zusätzlich muß das an der Rückseite der Ventilklappe herrschende Vakuum überwunden werden, so daß zwangsläufig bei einer Betätigung der Ventilklappe diese mit "Ruck" in die voll geöffnete Stellung geführt wird. Das hat operationstechnisch Nachteile. In vielen Fällen ist es nicht erwünscht, daß an der Saugmündung des Saugers immer das volle Vakuum zur Verfügung steht, sondern in bestimmten Einsatzfällen ist es sehr erstrebenswert, daß nur eine minimale Saugkraft am Saugrohrende einstellbar ist, wobei die Variation dieser Saugkraft nicht durch ventilartige Einrichtungen in der Vakuumleitung erfolgen soll, sondern vom Operateur im jeweiligen Einsatzfall von Hand einstellbar sein soll. Mit anderen Worten: Bei der bekannten Einrichtung gemäß der DE-B-2 950 323 ist eine Feineinstellung vom Operateur selbst nur schwer durchzuführen.

Im übrigen muß bei einem Sauger der vorstehend erläuterten Gattung berücksichtigt werden, daß während der Zeit, in der der Sauger nicht benutzt wird und die Saugleitung nach außen hin verschlossen ist, sich innerhalb der Saugleitung das von der Vakuummaschine erzielte Vakuum in vollem Umfang aufbauen kann, so daß nach einiger Ruhezeit des Saugers auf der Rückseite der Ventilklappe ein Vakuum in der Größenordnung von etwa 78 kPa (0,8 atm) herrscht. Durch das Ansaugen von Luft während des Betriebes des Saugers steht an der Saugermündung normalerweise nur ein Vakuum von 29 kPa (0,3 atm) zur Verfügung. Bei den bekannten Saugern wird also beim Öffnen des Ventils sofort das volle Vakuum wirksam, was in vielen Operationsfällen außerordentlich unerwünscht ist, da die Saugleistung zu groß ist.

Schließlich ist bei dem Sauger gemäß der DE-B-2 950 323 der Raum, in dem die Ventilklappe wirksam ist, nicht zugänglich, d. h. Korrekturen einer Falschlage der Ventilklappe sind nur sehr schwer möglich. Durch die angegossene Gelenkverbindung ist keine sichere Führung der Ventilklappe gewährleistet. Außerdem bildet sich unter dem in den Ventilraum vorstehenden Rohrteil des Saugrohres ein Raum, in dem sich Schmutzpartikel und unkontrollierbare Sekretreste und Knochensplitterchen ansammeln können, die u. U. zur Fehlfunktion der Ventilklappe führen.

In der FR-A-1 426 230 wird ein gattungsfremder Operationssauger beschrieben, bei welchem zum Öffnen oder Schließen ein federbelasteter Handgriff vorgesehen wird, der mit einem Ventilstößel verbunden ist, der den Querschnitt des Saugrohres verschließen oder öffnen kann. Zusätzlich ist eine Feineinstellung über eine Stellschraube möglich. Dieser Operationssauger hat den Nachteil, daß im Betrieb die Hand des Chirurgen durch die hohe Rückstellkraft der Feder im Handgriff belastet wird und daß zusätzlich das Drehventil eingestellt werden muß, so daß insgesamt eine ungünstige Gestaltung erreicht wird, da die Drehbewegung nicht ohne Zuhilfenahme der anderen Hand durchgeführt werden kann. Der Sauger kann also in vollem Umfang vom Operateur selbst nicht vollständig bedient werden, sondern ggf. ist eine Bedienungsperson erforderlich.

Der Erfindung liegt die Aufgabe zugrunde, einen Operationssauger zu schaffen, bei dem die Ventileinrichtung möglichst leichthängig und einhändig zu bedienen ist, so daß dadurch die Hand des Operaturs bei der Betätigung des Saugers entlastet wird und außerdem eine Feindosierung an der Saugermündung möglich ist, wobei trotzdem ein Ansammeln von Sekretresten und Knochensplitterchen im Bereich der Ventilklappe vermieden wird.

Diese der Erfindung zugrundeliegende Aufgabe wird durch den kennzeichnenden Teil des Hauptanspruches gelöst.

Der Hauptanspruch schlägt eine Einrichtung vor, bei welcher die beiden Saugrohrabschnitte zumindest im oberen Bereich über ein ein elastisches Mittel verbunden sind, das die Betätigung der Ventilklappe zumindest in der Anfangsphase der Öffnungsbewegung der Ventilklappe muß nur gegen das auf der Ventilklappenrückseite wirkende Vakuum erfolgen, wobei diese Öffnungskraft, die erforderlich ist, durch eine in der elastischen Wandung inkorporierte zusätzliche Kraft unterstützt wird. Erst wenn eine Teilöffnung der Ventilklappe erfolgt ist, auf die Rückseite der Ventilklappe also nicht mehr das volle Vakuum wirksam ist, muß die Öffnungsbewegung gegen die elastische Kraft der Gehäusewandung weiter durchgeführt werden. Dies gibt dem Operateur die Möglichkeit, bereits in der Anfangsphase der Öffnungsbewegung eine Teilöffnung der Klappe

herbeizuführen, so daß dadurch zwangsläufig eine Feindosierung an der Saugerspitze erreicht wird.

Vorteilhafte Ausführungsformen der Erfindung sind in den Unteransprüchen erläutert.

In der nachfolgender Beschreibung wird eine Ausführungsform der Erfindung beschrieben. Hierbei zeigen

Fig. 1 eine Ausführungsform der Erfindung, bei welcher die Ventilklappe in der Ruhestellung steht, d. h. auf die Rückseite der Ventilklappe kein Vakuum wirksam ist,

Fig. 2 die Ausführungsform nach Fig. 1, bei auf der Rückseite wirkendem Vakuum und

Fig. 3 die Ausführungsform gemäß Fig. 1 bei voll geöffneter Ventilklappe.

Fig. 4 zeigt den in die elastische Wandung einsetzbaren Betätigungsstößel in Draufsicht.

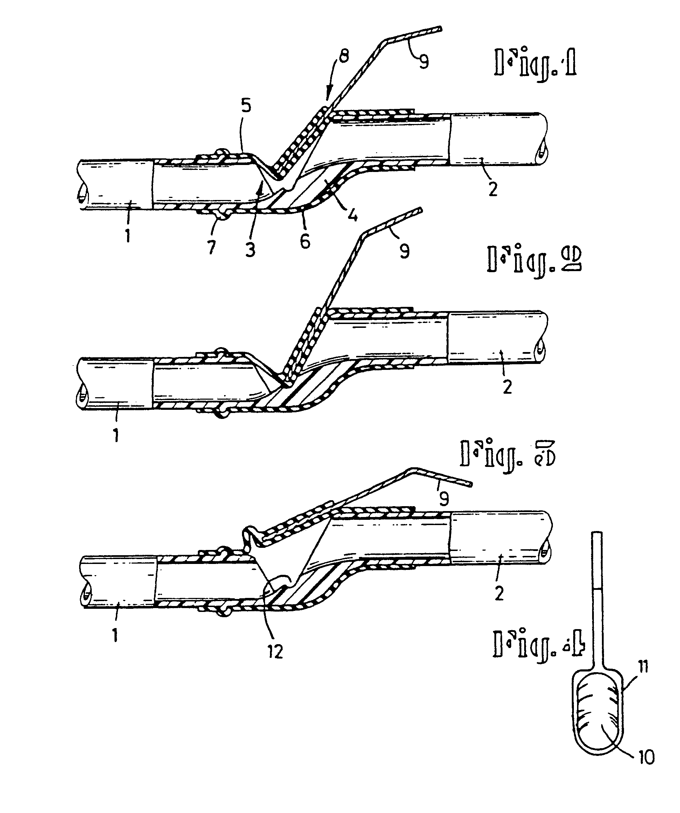

In den Fig. 1 bis 3 sind zwei Rohrstücke 1 und 2 dargestellt, die das eigentliche Saugrohr bilden, wobei die Mündung, d. h. also der Saugerkopf, in der Zeichnung nicht dargestellt ist. Die beiden Rohrstücke 1 und 2 sind stufenförmig gegeneinander versetzt und weisen zumindest an ihrer oberseite einen Ausschnitt 3 auf. Die Rohre können dabei einteilig in einem Fertigungsprozeß so hergestellt werden, daß dieser Ausschnitt vorgesehen ist, wobei die Bodenseite der Rohre dann durch eine mit den Rohrstücken 1 und 2 einteilig ausgebildete Verdickung 4 gebildet ist.

Die beiden Rohrstücke 1 und 2 werden über ein Gehäuse 5 miteinander verbunden, das aus einem Gummischlauchstück 6 besteht und beispielsweise über entsprechende Formgebungen 7 oder zusätzliche Rohrschellen an den Rohrstücken 1 und 2 festgelegt werden kann.

An der oberseite des dargestellten Gehäuses, d. h. dem Bereich, der im Bereich des Ausschnittes 3 liegt, ist eine Einstecktasche 8 vorgesehen, in die bei dem dargestellter Ausführungsbeispiel ein Bedienungshebel 9 eingesetzt ist. Natürlich kann der Bedienungshebel 9 auch einteilig aus dem Gummischlauchstück 6 ausgeformt oder einteilig in diesen eingegossen sein

Der Bedienungshebel 9 kann aus Metall bestehen und auswechselbar sein, in gleicher Weise aber auch aus Kunststoff hergestellt werden, so daß bei der Entsorgung der Ausbau des Bedienungshebels nicht erforderlich ist.

Anstelle der dargestellten Einstecktasche und der dargestellten Ausbildung der Oberseite des Gehäuses kann dieses in dem Bereich, wo die Ventilklappe gebildet werden soll, als Kugelkörper oder Teilkugelkörper ausgebildet sein.

Die Formgebung, Materialwahl und damit das elastische Rückstelvermögen des Gummischlauchstückes 6 ist derart, daß im Ruhezustand, d. h, bei in den Rohrstucken 1 und 2 nicht wirksamen Vakuum, die durch den in der Einstecktasche 8 befindlichen Teil des Bedienungshebels gebildete Ventilklappe in einer Stellung steht, in der diese Klappe die Mündung des Rohrstückes 2 nicht verschließt, sondern in dieser Ruhestellung ist die Rohrmündung 2 teilweise offen, d. h. nicht durch die Ventilklappe verschlossen Anstelle, daß die Verdickung 4 einteilig aus den beiden Rohrstücken 1 und 2 ausgeformt ist, ist es natürlich auch möglich, die Verdickung 4 im Gummischlauchstück 6 vorzusehen und die Rohrstücke 1 und 2 als getrennte Bauteile einzusetzen.

Wird nunmehr im Rohrstück 2 ein Vakuum erzeugt, schließt sich aufgrund der auf die Rückseite der Ventilklappe wirkenden Saugkraft die Ventilklappe, d. h. sie verschließt die Mündung des Rohrstückes 2.

Zum Öffnen der Ventilklappe wird, wie dies Fig. 3 verdeutlicht der Bedienungshebel 9 so weit niedergedrückt, daß er das gummielastische Gehäuse oberhalb des Auschnittes 3 anhebt, so daß damit eine freie Durchflußöffnung für das Vakuum erzielt wird.

Aus einem Vergleich der Darstellung 1 bis 3 ist ersichtlich, daß zum Öffnen der Ventilklappe aus der in Fig. 2 dargestellten Lage in die in Fig. 3 dargestellte Lage, zuerst die Saugkraft des Vakuums überwunden werden muß, die im Rohrstück 2 herrscht, wobei zum überwinden dieser Saugkraft die Elastizität des Gehäuses beiträgt, nämlich das Gehäuse selbst ist ja bestrebt, den Bedienungshebel in die in Fig. 1 dargestellte Lage anzuheben.

In Fig. 4 ist der Bedienungshebel dargestellt, und es ist erkennbar, daß der in der Einstecktasche 8 einsteckbare Teil 10 des Bedienungshebels gewölbt ausgebildet ist und einen ebenen Außenrand 11 aufweist, wobei die Wölbung kugelabschnittförmig ist, aber langgestreckt und damit oval, so daß damit ein möglichst großer freier Durchflußquerschnitt in der in Fig. 3 dargestellten Saugstellung des Bedienungshebels ermöglicht wird. Der Bedienungshebel kann aber auch stiftförmig gestaltet sein, so daß dadurch bei seinem Anheben automatisch die gewünschte Verformung des Gehäuses erfolgt.

Bei 12 ist eine umlaufende Vertiefung in der Verdickung 4 erkennbar, in die sich der untere Teil des ebenen Außenrandes 11 des Bedienungshebels 9 einlegt. Diese Verschlußstellung zeigt Fig. 2.

**Patentansprüche**

1. Operationssauger zum Absaugen von Flüssigkeiten bei chirurgischen Eingriffen mit einem von Hand in das Operationsfeld einzuführenden Saugrohr, das aus zwei über ein Gehäuse (5) verbundenen Rohrstücken (1, 2) besteht, dessen eines (2) ein vorzugsweise schräg abgeschnittenes Rohrende in dem Gehäuse (5) aufweist, und mit einem rückschlagventilartigen Unterbrechungsorgan für

den Saugstrom, das durch die das Saugrohr haltende Hand bedienbar ist und im Inneren des Gehäuses eine dem vorzugsweise schräg abgeschnittenen Rohrende angepaßte, mit einem nach außen vorstehenden Bedienungshebel (9) versehene Ventilklappe aufweist, der das Rohrende als Ventil sitz dient, dadurch gekennzeichnet, daß das Gehäuse (5) wenigstens an seiner Oberseite elastisch ausgebildet ist und an seiner Innenseite im Bereich des elastischen Bereiches als Ventilklappe der Rohröffnung angepaßt und so ausgebildet bzw. dimensioniert ist, daß im Ruhezustand, d. h. vakuumlosen Zustand, die Ventilklappe die zu verschließende Rohröffnung nicht voll verschließt.

2. Sauger nach Anspruch 1, dadurch gekennzeichnet, daß das elastische Gehäuseteil (5) eine nach außen offene Einstecktasche (8) für den Bedienungshebel aufweist.

3. Sauger nach Anspruch 1, dadurch gekennzeichnet, daß die beiden Rohrstücke (1, 2) innerhalb des Gehäuses in ihrem unteren Bereich einteilig ausgebildet und in ihrer Längsachse stufenförmig gegeneinander versetzt sind und in ihrem oberen Bereich einen Ausschnitt (3) aufweisen zur Aufnahme der Ventilklappe.

4. Sauger nach Anspruch 2, dadurch gekennzeichnet, daß das die beiden Rohrstücke überbrückende Gehäuse (5) aus einem Gummischlauchstück (6) besteht, an dem ausgeformt an seiner Oberseite die Einstecktasche (8) vorgesehen ist.

5. Sauger nach Anspruch 1, dadurch gekennzeichnet, daß das Gehäuse (5) durch einen aus gummielastischem Werkstoff bestehenden und um die beiden Rohrstücke (1, 2) verschließbaren Mantel besteht.

6. Sauger wenigstens nach Anspruch 1 und 2, dadurch gekennzeichnet, daß der Bedienungshebel (9) in seinem innerhalb der Tasche (8) bzw. des elastischen Teiles des Gehäuses (5) befindlichen Bereich kugelabschnittförmig (bei 10) mit nach oben gewölbter Kugelfläche ausgebildet ist und einen ebenen Außenrand (11) aufweist.

7. Sauger nach Anspruch 1, dadurch gekennzeichnet, daß der Bedienungshebel (9) aus Kunststoff besteht.

8. Sauger nach Anspruch 1, dadurch gekennzeichnet, daß der Bedienungshebel nur stiftförmig ausgebildet ist

9. Sauger nach Anspruch 1, dadurch gekennzeichnet, daß der Bedienungshebel als Zugelement an der Oberseite des die Ventilklappe bildenden Gehäuse.

one (2) of which pipe sections (1, 2) has in the housing (5) a pipe end which is preferably cut off at an angle, and further comprising a non-return valve-type interruption member for the suction flow, which is operable by the hand holding the suction pipe and comprises inside the housing a valve flap which is conformed to the preferably slopingly cut-off pipe end, is provided with an outwardly projecting operating lever (9) and has the pipe end serving as a valve seat, characterized in that the housing (5) is constructed resiliently at least at its top, is adapted on the inside in the region of the resilient area to be the valve flap for the pipe opening and is so constructed or dimensioned that in the rest state, i.e. the vacuum-less state, the valve flap does not completely close the pipe opening to be closed.

2. A suction device according to claim 1, characterized in that the resilient housing part (5) comprises an outwardly open insert pocket (8) for the operating lever.

3. A suction device according to claim 1, characterized in that the two pipe sections (1, 2) are constructed in one piece inside the housing in their lower area and are staggered with respect to each other along their longitudinal axes in the manner of steps and comprise an indentation (3) in their upper area for receiving the valve flap.

4. A suction device according to claim 2, characterized in that the housing (5) connecting the two pipe sections consists of a rubber hose piece (6), out of the top of which is formed the insert pocket (8).

5. A suction device according to claim 1, characterized in that the housing (5) consists of a jacket consisting of rubber-elastic material and closable around the two pipe sections (1, 2).

6. A suction device according to at least claim 1 and claim 2, characterized in that the operating lever (9) is formed, in its area inside the pocket (8) or the resilient part of the housing (5), in the manner of a spherical segment (at 10) with an upwardly domed spherical surface and comprises a flat outer edge (11).

7. A suction device according to claim 1, characterized in that the operating lever (9) consists of plastics material.

8. A suction device according to claim 1, characterized in that the operating lever is only constructed in a pin-shape.

9. A suction device according to claim 1, characterized in that the operating lever is constructed as a pull member at the top of the housing part forming the valve flap.

**Claims**

1. A medical suction device for sucking out fluids during surgical operations comprising a suction pipe to be introduced into the operation area by hand, which suction pipe consists of two pipe sections (1, 2) connected via a housing (5),

**Revendications**

1. Aspirateur chirurgical destiné à aspirer des liquides au cours d'interventions chirurgicales, comprenant un tube suceur qui peut être placé manuellement sur le site de l'opération, qui est composé de deux tronçons de tubes (1, 2) réunis par un corps (5), et dont l'un (2) présente une extrémité de tube de préférence coupée en oblique à l'intérieur du corps (5) et un organe d'interruption du courant d'aspiration, du type clapet anti-retour, qui peut être manoeuvré par la main qui tient le tube d'aspiration, et qui présente à l'intérieur du corps un clapet adapté à l'extrémité du tube qui est de préférence coupée en oblique, et muni d'un levier de manoeuvre (9) qui fait saillie à l'extérieur, l'extrémité du tube servant de siège de clapet, caractérisé en ce que le corps (5) est de constitution élastique, du moins sur son côté supérieur, tandis que, sur son côté intérieur, dans la région de la zone élastique, il est adapté pour servir de clapet pour fermer l'ouverture du tube, et constitué ou dimensionné de telle manière qu'à l'état de repos, c'est-à-dire dans l'état sans dépression, le clapet ne ferme pas entièrement l'ouverture du tube qu'il s'agit d'obturer.

2. Aspirateur selon la revendication 1, caractérisé en ce que la partie élastique (5) du boîtier présente une poche réceptrice (8) s'ouvrant vers l'extérieur et destinnée à recevoir le levier de manoeuvre.

3. Aspirateur selon la revendication 1, caractérisé en ce que les deux tronçons de tubes (1, 2) sont d'une seule pièce dans leur région intérieure à l'intérieur du corps et sont décalés en escalier dans leur axe longitudinal et présentent, dans leur région supérieure une échancrure (3) destinée à recevoir le clapet.

4. Aspirateur selon la revendication 2, caractérisé en ce que le corps (5) qui forme pont sur les deux tronçons de tubes (1,2) est constitué par un morceau de tuyau en caoutchouc (6) sur lequel la poche réceptrice (8) est venue de moulage, sur sa face supérieure.

5. Aspirateur selon la revendication 1, caractérisé en ce que le corps (5) est constitué par une enveloppe composée d'une matière possédant l'élasticité du caoutchouc et qui peut être montée à joint étanche sur la périphérie des deux tronçons de tubes,

6. Aspirateur selon au moins les revendications 1 et 2, caractérisé en ce que, dans sa région contenue à l'intérieur de la poche (8), ou de la partie élastique du corps (5), le levier de manoeuvre (9) est d'une configuration en calotte de sphère (en 10), avec une surface sphérique bombée vers le haut et présente un bord extérieur plan (11).

7. Aspirateur selon la revendication 1, caractérisé en ce que le levier de manoeuvre (9) est fait de matière plastique.

8. Aspirateur selon la revendication 1, caractérisé en ce que le levier de manoeuvre présente seulement la forme d'une tige.

9. Aspirateur selon la revendication 1, caractérisé en ce que le levier de manoeuvre est constitué par un élément de traction formé sur la face supérieure du corps qui forme le clapet.

Fig. 1

Fig. 2

Fig. 3

Fig. 4